# EUROPEAN PATENT SPECIFICATION

(11) **EP 3 165 213 B1**
(45) Date of publication and mention of the grant of the patent: **06.05.2020**
(21) Application number: 16197162.7
(22) Date of filing: 03.11.2016
(51) Int. Cl.: A61H 9/00, A61H 31/00, A61M 16/00

(54) **DYNAMIC CONTROL OF RESPIRATORY THERAPY DEVICES**
DYNAMISCHE STEUERUNG VON ATEMTHERAPIEVORRICHTUNGEN
COMMANDE DYNAMIQUE DE DISPOSITIFS DE THÉRAPIE RESPIRATOIRE

(30) Priority: 06.11.2015 US 201562251735 P
(43) Date of publication of application: 10.05.2017
(73) Proprietor: Hill-Rom Services Pte. Ltd., Singapore 768923 (SG)
(72) Inventor: WILLIAMS, Joshua, A, West Harrison, IN Indiana 47060 (US); ROSSINI, Mary, Jane, Fridley, MN Minnesota 55432 (US)
(74) Representative: Findlay, Alice Rosemary

(56) References cited:
- WO-A1-2011/048524
- WO-A1-2013/144827
- US-A1- 2011 196 251
- US-A1- 2013 199 534
- US-B2- 7 115 104

## Description

The present disclosure relates generally to respiratory therapy systems, and more particularly to respiratory therapy systems that include dynamic therapy control of a respiratory therapy device for administering respiratory therapy.

Respiratory therapy systems are used to treat a variety of diseases, such as cystic fibrosis, emphysema, asthma and chronic bronchitis, to remove excess mucus that collects in the lungs. Some of the therapies include manual percussion techniques of chest physiotherapy and mechanical insufflation/exsufflation techniques. To bypass dependency on a caregiver to provide respiratory therapy, high-frequency chest wall oscillation (HFCWO) devices and mechanical insufflation-exsufflation (MIE) devices have been developed. The HFCWO devices have been developed to deliver HFCWO therapy to a patient's torso to promote airway mucus clearance by generating rapidly oscillating externally powered cough like air flows and pressures in the airways of a patient. The MIE devices have been developed to deliver MIE therapy to a patient by artificially stimulating a cough internally to clear airway secretions.

The device most widely used to produce HFCWO therapy is THE VEST™ airway clearance system available from Hill-Rom Company, Inc. An example of such a system is shown and described in U.S. Pat. No. 7,115,104. Another example of a device used for HFCWO therapy is shown and described in U.S. Pat. App. Pub. No. 2014/0012167 A1. THE VEST™ device applies pneumatic pulses to a patient's chest via a bladder of a vest worn by the patient and the device shown in U.S. Pat. App. Pub. No. 2014/0012167 A1 applies mechanical pulses to a patient's chest using a set of mechanical percussors that are mounted to a vest worn by the patient. Further, an example of a mechanical insufflation/exsufflation (MIE) airway clearance apparatus is shown and described in U.S. Pat. No. 8,539,952. The MIE airway clearance apparatus cyclically applies positive and negative pressure to a patient's airway via a mask or mouthpiece.
Further examples of prior art devices may be found in WO 2013/144827, US 2013/199534, WO2011/048524 and US2011/196251.

The invention is defined by the claims.

The invention will now be further described by way of example with reference to the accompanying drawings, in which:
Fig. 1 is a block diagram showing a system for dynamic therapy control of a respiratory therapy device usable to administer a respiratory therapy session;
Fig. 2 and is a block diagram of an environment that may be established by the respiratory therapy device of Fig. 1;
Figs. 3 and 4 are a flow diagram of an embodiment of a method for administering a respiratory therapy treatment session that may be executed by the respiratory therapy device of Figs. 1 and 2; and
Figs. 5 and 6 are a flow diagram of an embodiment of a method for diagnostic monitoring of the respiratory therapy device of Figs. 1 and 2 that may be executed by the respiratory therapy device of Figs. 1 and 2 and/or the computing device of Fig. 1.

The term "air" is used herein broadly to include regular or ambient air, medical air, nitrogen, oxygen, and any other breathable, as well as non-breathable, gas available in any location including a hospital or healthcare facility.

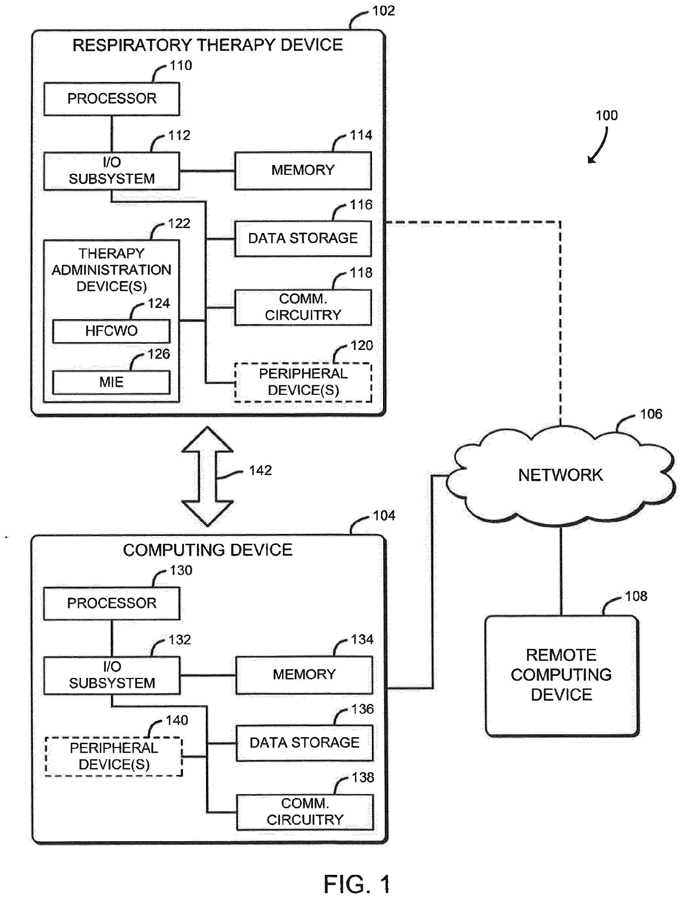

An embodiment of a system 100 for dynamic therapy control of a respiratory therapy device 102, such as a pneumatic high frequency chest wall oscillation (HFCWO) device such as THE VEST™ airway clearance system available from Hill-Rom Company, Inc. and/or a HFCWO device having mechanical percussors, is illustrated diagrammatically in Fig. 1. The illustrative respiratory therapy device 102 is usable to administer a respiratory therapy session to effectively clear mucous or induce deep sputum removal from the lungs of a patient wearing the respiratory therapy device 102. As shown in Fig. 1, the respiratory therapy device 102 is communicatively coupled to a computing device 104 via a communication channel 142, which may be established by a wired connection or a wireless connection.

For example, in one such embodiment, the computing device 104 may be a mobile computing device communicatively coupled to the respiratory therapy device 102 via a short-range wireless connection (e.g., Bluetooth®, Bluetooth® Low Energy (BLE), near-field communication (NFC), and/or any other short-ranged wireless communication technology). As shown in the illustrative system 100, the computing device 104 may be further communicatively coupled to a remote computing device 108. Additionally or alternatively, in some embodiments, the respiratory therapy device 102 may be communicatively coupled to the remote computing device 108. It should be appreciated that, in such embodiments, the system 100 may not include the computing device 104.

In use, the respiratory therapy device 102 receives an input indicating a parameter such as total number of therapy cycles to be administered during a therapy session. Additionally, the respiratory therapy device 102 receives a number of other therapy parameters for each of the received therapy cycles in some embodiments. The other therapy parameters identify data capable of defining a function of the respiratory therapy device 102 during a cycle of a therapy session. For example, the therapy parameters may include an oscillating compressive force, an oscillation frequency, an oscillation frequency sweep range, an oscillation frequency step range, a duration (i.e., an amount of time to perform the therapy parameters corresponding to a particular cycle), a bias line pressure, an insufflation pressure, an exsufflation pressure, etc.

In some embodiments, the total number of therapy cycles and the therapy parameters for each of the therapy cycles may be directly input into the respiratory therapy device 102, such as by an operator (e.g., a user, a physician, etc.) of the respiratory therapy device 102, via one or more input controls of the respiratory therapy device 102. Alternatively, in some embodiments, the therapy parameters may be input by the operator via the computing device 104 communicatively coupled to the respiratory therapy device 102. In such embodiments, the computing device 104 may include an application capable of being executed on the computing device 104 and performing at least a portion of the functions described herein. Additionally or alternatively, in such embodiments wherein the respiratory therapy device 102 receives the inputs from the computing device 104, the inputs may be stored local to the computing device 104 and/or the respiratory therapy device 102. Subsequent to administering the therapy session, the operator of the respiratory therapy device 102 may be prompted to save the therapy parameters of the therapy session for later recall, such as to a designated preset, at the respiratory therapy device 102 or the computing device 104, depending on the embodiment.

Additionally, in some embodiments, the respiratory therapy device 102 may be capable of performing a diagnostic check, or otherwise monitoring diagnostics of the respiratory therapy device 102 (i.e., repeatedly performing the diagnostic check at a predetermined interval). In such embodiments, the respiratory therapy device 102 may check one or more of the functionality, settings, and measurable characteristics of the respiratory therapy device 102. For example, the respiratory therapy device 102 may be configured to check a battery life, a number of hours of use, as well as test any other functions or measure other characteristics of the respiratory therapy device 102. In such embodiments wherein the diagnostic monitoring is being reported to the user via the computing device 104, the respiratory therapy device 102 may be configured to transmit the results of the diagnostic check(s) to the computing device 104.

Based on the results of the diagnostic monitoring, the respiratory therapy device 102 may provide a notification (e.g., a visual notification, an audible notification, etc.) to the operator of the respiratory therapy device 102 as to whether the diagnostics resulted in a potential issue having been detected or not. Upon receiving a notification indicative of an issue, the operator may then address the issue by, depending on the embodiment, reporting the issue(s), troubleshooting the issue(s), and/or contacting support staff with regards to the issue(s) either directly from the respiratory therapy device 102 and/or via the computing device 104 communicatively coupled to the respiratory therapy device 102.

The respiratory therapy device 102 may be embodied as any type of respiratory physiotherapy and/or airway mucus clearance device capable of performing the functions described herein. As shown in Fig. 1, the illustrative respiratory therapy device 102 includes a processor 110, an input/output (I/O) subsystem 112, a memory 114, a data storage device 116, communication circuitry 118, and one or more therapy administration devices. Of course, the respiratory therapy device 102 may include other or additional components, such as those commonly found in a respiratory therapy device (e.g., one or more input/output peripheral devices), in other embodiments. Additionally, in some embodiments, one or more of the illustrative components may be incorporated in, or otherwise form a portion of, another component. For example, the memory 114, or portions thereof, may be incorporated in the processor 110 in some embodiments. Further, in some embodiments, one or more of the illustrative components may be omitted from the respiratory therapy device 102.

The processor 110 may be embodied as any type of processor capable of performing the functions described herein. For example, the processor 110 may be embodied as a single or multi-core processor(s), digital signal processor, microcontroller, or other processor or processing/controlling circuit. Similarly, the memory 114 may be embodied as any type of volatile or non-volatile memory or data storage capable of performing the functions described herein. In operation, the memory 114 may store various data and software used during operation of the respiratory therapy device 102, such as operating systems, applications, programs, libraries, and drivers.

The memory 114 is communicatively coupled to the processor 110 via the I/O subsystem 112, which may be embodied as circuitry and/or components to facilitate input/output operations with the processor 110, the memory 114, and other components of the respiratory therapy device 102. For example, the I/O subsystem 112 may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations. In some embodiments, the I/O subsystem 112 may form a portion of a system-on-a-chip (SoC) and be incorporated, along with the processor 110, the memory 114, and other components of the respiratory therapy device 102, on a single integrated circuit chip.

The data storage device 116 may be embodied as any type of device or devices configured for short-term or long-term storage of data such as, for example, memory devices and circuits, memory cards, hard disk drives, solid-state drives, or other data storage devices. It should be appreciated that the data storage device 116 and/or the memory 114 (e.g., the computer-readable storage media) may store various data as described herein, including operating systems, applications, programs, libraries, drivers, instructions, etc., capable of being executed by a processor (e.g., the processor 110) of the respiratory therapy device 102.

The communication circuitry 118 may be embodied as any communication circuit, device, or collection thereof, capable of enabling communications between the respiratory therapy device 102 and other computing devices (e.g., the computing device 104, the remote computing device 108, etc.) over a network (e.g., the network 106) or over a wired or wireless communication technology. Accordingly, the communication circuitry 118 may be configured to use any one or more communication technologies (e.g., wireless or wired communication technologies) and associated protocols (e.g., Ethernet, Bluetooth®, Wi-Fi®, WiMAX, LTE, 5G, etc.) to effect such communication.

The illustrative therapy administration devices 122 include a high-frequency chest wall oscillation (HFCWO) device 124 and a mechanical insufflation-exsufflation (MIE) device 126. The HCFWO device 124 may include various components not shown for clarity of the description, such as a chest wall oscillator to generate an HFCWO therapy session to the patient. To do so, the respiratory therapy device 102 may be operationally coupled (e.g., via one or more hoses) to a garment (e.g., a vest, a chest rap, etc.) worn on a torso of a patient to deliver the HFCWO therapy session to the patient. The HFCWO therapy session may include application to the garment of one or more of the therapy parameters discussed previously. The MIE device 126 may include various components not shown for clarity of the description, such as a mechanical insufflator/exsufflator to deliver an MIE therapy session to the patient by way of a mask (e.g., via one or more hoses) worn by the patient (i.e., coupled to the patient's airway to deliver the MIE therapy session to the patient) or a mouthpiece or a nasal cannula. The MIE therapy session may include application to the patient interface of one or more of the therapy parameters discussed previously.

In some embodiments, the respiratory therapy device 102 may additionally include one or more peripheral devices 120. The peripheral devices 120 may include any number of additional peripheral or interface devices, such as buttons, speakers, microphones, additional storage devices, and so forth. The particular type of peripheral devices included in the peripheral devices 120 may depend on, for example, a model or purpose of the respiratory therapy device 102. Additionally or alternatively, the peripheral devices 120 may include one or more ports, such as a USB port, for example, for connecting external peripheral devices to the respiratory therapy device 102.

The computing device 104 may be embodied as any type of computation or computing device capable of performing the functions described herein, including, without limitation, a computer, a mobile computing device (e.g., smartphone, tablet, laptop, notebook, wearable, etc.), a server (e.g., stand-alone, rack-mounted, blade, etc.), a network appliance (e.g., physical or virtual), a web appliance, a distributed computing system, a processor-based system, and/or a multiprocessor system. Similar to the illustrative respiratory therapy device 102, the computing device 104 may include a processor 130, an I/O subsystem 132, a memory 134, a data storage device 136, and communication circuitry 138, as well as, in some embodiments, one or more peripheral devices. As such, further descriptions of the like components are not repeated herein with the understanding that the description of the corresponding components provided above in regard to the respiratory therapy device 102 applies equally to the corresponding components of the computing device 104.

The remote computing device 108 may be embodied as any type of computing device capable of performing the functions described herein, such as, without limitation, a server (e.g., stand-alone, rack-mounted, blade, etc.), a network appliance (e.g., physical or virtual), a high-performance computing device, a web appliance, a distributed computing system, a computer, a processor-based system, a multiprocessor system, and/or a mobile computing device (e.g., a smartphone, a tablet computer, a laptop computer, a notebook computer, a wearable computing device, etc.). Similar to the illustrative respiratory therapy device 102, the remote computing device 108 may include a processor, an I/O subsystem, a memory, a data storage device, and/or communication circuitry, which are not shown for clarity of the description. As such, further descriptions of the like components are not repeated herein with the understanding that the description of the corresponding components provided above in regard to the respiratory therapy device 102 applies equally to the corresponding components of the remote computing device 108.

The network 106 may be embodied as any type of wired or wireless communication network, including cellular networks, such as Global System for Mobile Communications (GSM) or Long-Term Evolution (LTE), telephony networks, digital subscriber line (DSL) networks, cable networks, local or wide area networks, global networks (e.g., the Internet), or any combination thereof. It should be appreciated that the network 106 may serve as a centralized network and, in some embodiments, may be communicatively coupled to another network (e.g., the Internet). Accordingly, the network 106 may include a variety of network devices (not shown), virtual and physical, such as routers, switches, network hubs, servers, storage devices, compute devices, etc., as needed to facilitate communication between the respiratory therapy device 102 and the remote computing device 108 and/or the respiratory therapy device 102 and the computing device 104.

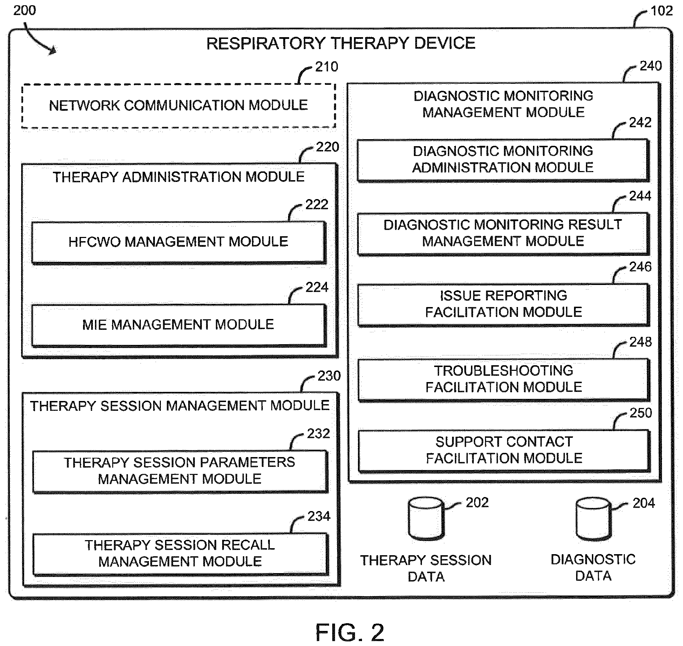

Referring now to Fig. 2, in an illustrative embodiment, the respiratory therapy device 102 establishes an environment 200 during operation. The illustrative environment 200 includes a network communication module 210, a therapy administration module 220, a therapy session management module 230, and a diagnostic monitoring management module 240. Each of the modules, logic, and other components of the environment 200 may be embodied as hardware, software, firmware, or a combination thereof. For example, each of the modules, logic, and other components of the environment 200 may form a portion of, or otherwise be established by, the processor 110, the communication circuitry 118, and/or other hardware components of the respiratory therapy device 102. As such, in some embodiments, one or more of the modules of the environment 200 may be embodied as circuitry or a collection of electrical devices (e.g., network communication circuitry 210, therapy administration circuitry 220, therapy session management circuitry 230, diagnostic monitoring management circuitry 240, etc.).

In the illustrative environment 200, the respiratory therapy device 102 includes therapy session data 202 and diagnostic data 204, each of which may be accessed by the various modules and/or sub-modules of the respiratory therapy device 102. It should be appreciated that the respiratory therapy device 102 may include other components, sub-components, modules, sub-modules, and/or devices commonly found in a computing node, which are not illustrated in FIG. 2 for clarity of the description.

The network communication module 210 is configured to facilitate inbound and outbound network communications (e.g., network traffic, network packets, network flows, etc.) to and from the respiratory therapy device 102. To do so, the network communication module 210 is configured to receive and process network packets from other computing devices (e.g., the computing device 104, the remote computing device 108, and/or other computing device(s) communicatively coupled via the network 106). Additionally, the network communication module 210 is configured to prepare and transmit network packets to another computing device (e.g., the computing device 104, the remote computing device 108, and/or other computing device(s) communicatively coupled via the network 106). Accordingly, in some embodiments, at least a portion of the functionality of the network communication module 210 may be performed by the communication circuitry 118.

The therapy administration module 220 is configured to administer a therapy session. To do so, the illustrative therapy administration module 220 includes an HFCWO management module 222 and an MIE management module 224. The HFCWO management module 222 is configured to interpret any inputs (i.e., the number of therapy cycles and the therapy parameters for each cycle) received at the respiratory therapy device 102 and administer an HFCWO therapy session based on one or more of the received inputs. The MIE management module 224 is configured to interpret any inputs (i.e., the number of therapy cycles and the therapy parameters for each cycle) received at the respiratory therapy device 102 and administer an MIE therapy session based on one or more of the received inputs. In some embodiments, the one or more of the inputs received at the respiratory therapy device 102 may be stored in the therapy session data 202.

It should be further appreciated that one or more of the inputs received at the respiratory therapy device 102 may be received by the respiratory therapy device 102 from a communicatively coupled computing device (e.g., the computing device 104 of Fig. 1). In other words, the computing device 104 may be configured to receive the input from the user and transmit the input to the respiratory therapy device 102. In such embodiments, the therapy administration module 220 is further configured to receive and interpret the inputs received from the computing device 104 to administer the therapy session.

The therapy session management module 230 is configured to manage the inputs (i.e., the number of therapy cycles and the therapy parameters for each cycle) of the therapy sessions. To do so, the illustrative therapy session management module 230 includes a therapy session parameters management module 232 and a therapy session recall management module 234. The therapy session parameters management module 232 is configured to manage the number of therapy cycles and the therapy parameters for each cycle for a particular therapy session. The therapy session recall management module 234 is configured to manage the recall of saved operational parameters. For example, the therapy session recall management module 234 may be configured to save and receive the number of therapy cycles and other therapy parameters for each cycle for a previously saved therapy session, such as from a designated present to which the previously saved therapy session has been assigned.

The diagnostic monitoring management module 240 is configured to manage diagnostic monitoring (i.e., periodic diagnostic checks performed at a predetermined series of intervals) of the respiratory therapy device 102. As described previously, the diagnostic monitoring may include checking the functionality, settings, or other measurable characteristics of the respiratory therapy device 102. Accordingly, the diagnostic monitoring management module 240 is further configured to check the functionality, settings, and/or other measurable characteristics of the respiratory therapy device 102.

To manage the diagnostic monitoring, the illustrative diagnostic monitoring management module 240 includes a diagnostic monitoring administration module 242, a diagnostic monitoring result management module 244, an issue reporting facilitation module 246, a troubleshooting facilitation module 248, and a support contact facilitation module 250.

The diagnostic monitoring administration module 242 is configured to administer which components, settings, functionality, etc., of the respiratory therapy device 102 are to be monitored via one or more diagnostic checks that may be performed at predetermined intervals. For example, the diagnostic monitoring administration module 242 may be configured to administer the monitoring of a remaining charge of a battery of the respiratory therapy device 102, hours of use or other usage metric for a particular component of the respiratory therapy device 102, etc.

It should be appreciated that, in some embodiments, the respiratory therapy device 102 may receive input from the user that indicates which components, settings, functionality, etc. of the respiratory therapy device 102 are to be monitored by the diagnostic monitoring administration module 242. It should be further appreciated that, in some embodiments, the indication of which components and/or conditions of the respiratory therapy device 102 to be monitored may be input from the user via the computing device 104 and transmitted to the respiratory therapy device 102 for execution thereon.

The diagnostic monitoring result management module 244 is configured to manage results of diagnostic monitoring of the respiratory therapy device 102, such as those diagnostic checks administered by the diagnostic monitoring administration module 242. In some embodiments, the diagnostic monitoring result management module 244 may be further configured to analyze the results of diagnostic monitoring to determine whether a potential issue has been detected. Additionally or alternatively, in some embodiments, the results of the diagnostic monitoring and/or a log of identified potential issues generated by the diagnostic monitoring result management module 244 may be stored in the diagnostic data 204.

It should be appreciated that, in some embodiments, the diagnostic monitoring result management module 244 may be further configured to transmit the results to the computing device 104 communicatively coupled to the respiratory therapy device 102. In such embodiments, the computing device 104 may be configured to perform the analysis of the results and/or one or more of the functions of the issue reporting facilitation module 246, the troubleshooting facilitation module 248, and the support contact facilitation module 250, each of which are described below. In other words, in such embodiments, one or more of the functions of the issue reporting facilitation module 246, the troubleshooting facilitation module 248, and the support contact facilitation module 250 may not be performed by (i.e., local to) the respiratory therapy device 102.

The issue reporting facilitation module 246 is configured to report any issues detected during the analysis of the results, such as may be detected during the analysis performed by the diagnostic monitoring result management module 244. For example, the issue reporting facilitation module 246 may be configured to report the issues directly to a manufacturer, a physician, or the user. The troubleshooting facilitation module 248 is configured to provide troubleshooting steps for any issues detected during the analysis of the results. For example, the troubleshooting facilitation module 248 may be configured to display a sequence of steps for resolving the detected issues via one or more output devices of the respiratory therapy device 102.

The support contact facilitation module 250 is configured to contact support for the respiratory therapy device 102 regarding any detected issues. For example, the support contact facilitation module 250 may be configured to generate an email to a predefined support email address of a manufacturer of the respiratory therapy device 102 and/or a physician of the user. In another example, the support contact facilitation module 250 may be configured to place a phone call or generate a fax to a phone number of the manufacturer of the respiratory therapy device 102 and/or the physician of the user. In some embodiments, the support contact facilitation module 250 may be configured to facilitate the contacting of support at the request of the user (i.e., on-demand), regardless of whether an issue has been detected.

As described previously, in some embodiments, one or more functions of the illustrative respiratory therapy device 102 of Fig. 2 may be performed by a communicatively coupled computing device (e.g., the computing device 104 of Fig. 1). In such embodiments, it should be appreciated that the computing device 104 may include one or more modules similar to those shown in the illustrative respiratory therapy device 102 of Fig. 2, as described in detail above, depending on which functions the computing device 104 is configured to perform. Accordingly, in such embodiments, the respiratory therapy device 102 and the computing device 104 may establish a communication channel (e.g., the communication channel 142 of Fig. 1) in which to transfer data therebetween, such that the computing device 104 can perform those particular functions.

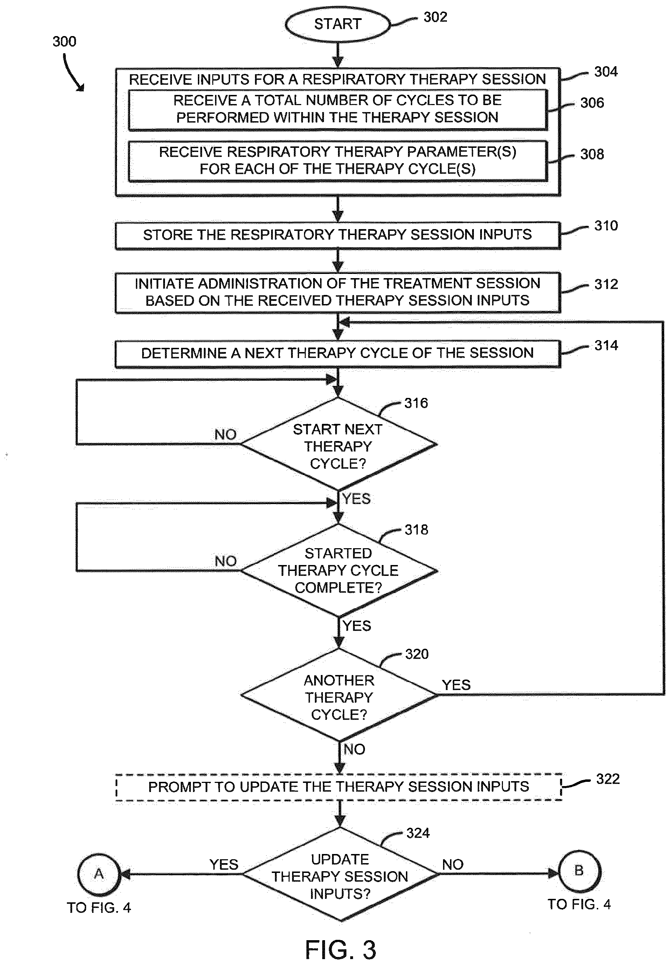

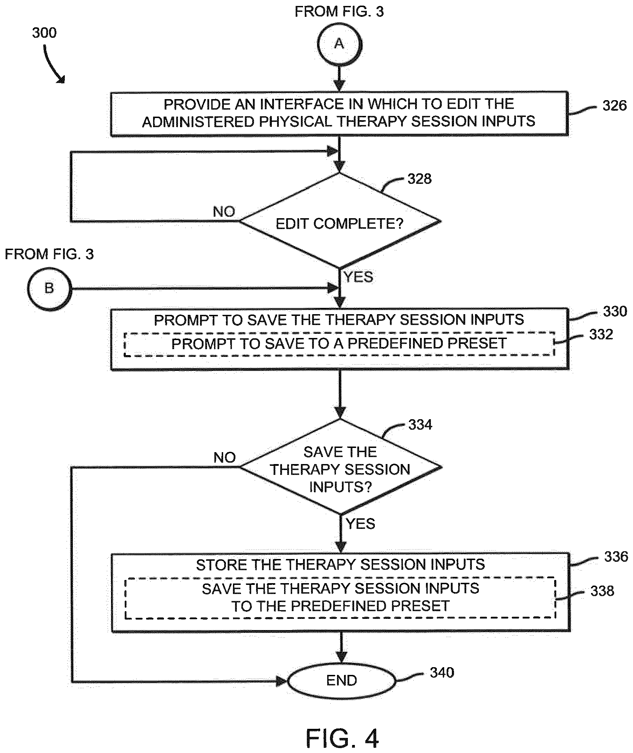

Referring now to Figs. 3 and 4, in use, the respiratory therapy device 102 may execute a method 300 for administering a respiratory therapy treatment session. The method 300 starts at step 302, which may be initiated by a user input, a timer, execution of an application, or any other action configured to initiate the administration of the respiratory therapy treatment session. In step 304, the respiratory therapy device 102 receives input for a respiratory therapy session. For example, in step 306, the respiratory therapy device 102 receives a total number of therapy cycles to be performed within the respiratory therapy session. Additionally, in step 308, the respiratory therapy device 102 receives one or more other respiratory parameters for each of the therapy cycles received in step 306.

In step 310, the respiratory therapy device 102 stores the respiratory therapy session inputs (i.e., the total number of therapy cycles and the other respective respiratory parameters). In step 312, the respiratory therapy device 102 initiates administration of the treatment session based on the received therapy session inputs. It should be appreciated that the initiation may be triggered by another input received from the user.

As described previously, it should be appreciated that, in some embodiments, the respiratory therapy session inputs may be directly input to one or more components of the respiratory therapy device 102. Additionally or alternatively, as also described previously, it should be further appreciated that in some embodiments the respiratory therapy session inputs may be received from a computing device (e.g., the computing device 104 of Fig. 1) communicatively coupled to the respiratory therapy device 102. Accordingly, it should be appreciated that, in such embodiments, one or more of the steps 304-312 may be performed by the computing device 104.

In step 314, the respiratory therapy device 102 determines a next therapy cycle of the therapy session (i.e., the first therapy cycle in the first iteration of the method 300). In step 316, the respiratory therapy device 102 determines whether to start the next therapy cycle. In some embodiments, the next therapy may be started based on a value of a timer (e.g., based on a run duration of a previously run therapy cycle) or by an input from the user.

If the next therapy cycle is to be started, the method 300 advances to step 318 to determine whether the therapy cycle started in step 316 has completed. If so, the method 300 advances to step 320 to determine whether there is another therapy cycle in the therapy session. If so, the method 300 returns to step 314 to determine the next therapy cycle of the therapy session; otherwise, the method 300 advances to step 322, wherein the respiratory therapy device 102 prompts to update the therapy session inputs.

It should be appreciated that the type of prompt may depend one which device (i.e., the respiratory therapy device 102 or the computing device 104) the update is to be provided to the user. For example, in embodiments wherein the user inputs the therapy session inputs directly into the respiratory therapy device 102, the prompt may be displayed or otherwise provided to the user at the respiratory therapy device 102. In another example, in embodiments wherein the user inputs the therapy session inputs into the computing device 104 coupled to the respiratory therapy device 102, the prompt may be displayed or otherwise provided to the user via the computing device 104.

In step 324, the respiratory therapy device 102 determines whether to update the therapy session inputs, such as may be based on an input received as a result of the prompt presented in step 322. For example, the user may determine at the end of the therapy session that the second cycle was not to their liking. In such an example, the user may indicate to the respiratory therapy device 102 or the computing device 104 as such. Accordingly, if the user provides an indication to update the therapy session inputs, the method 300 branches to step 326; otherwise, the method 300 branches to step 330, described below. In step 326, the respiratory therapy device 102 provides an interface in which the user may edit one or more of the administered physical therapy session inputs. As described previously, in some embodiments, the user interface for interacting with the user (e.g., receiving inputs, providing information, etc.) may be performed by one or more components of the computing device 104. Accordingly, it should be appreciated that, in such embodiments, one or more of the steps 324-336 may be performed by the computing device 104.

In step 328, the respiratory therapy device 102 determines whether the edit has been completed (i.e., the therapy session inputs have been updated). For example, the respiratory therapy device 102 may receive another input from the user indicating they have completed updating the physical therapy session inputs. If the respiratory therapy device 102 determines the edit has been completed, the method 300 proceeds to step 330, wherein the respiratory therapy device 102 prompts the user to save the therapy session inputs. For example, in some embodiments, in step 332, the respiratory therapy device 102 may prompt the user to save the therapy session inputs to a predefined preset that can be selected for recall of the therapy session at a future point in time.

In step 334, the respiratory therapy device 102 determines whether to save the therapy parameters, such as may be based on an input received as a result of the prompt presented in step 332. If not, the method 300 proceeds to step 340, wherein the method 300 terminates; otherwise, the method 300 advances to step 336, wherein the respiratory therapy device 102 stores the therapy session inputs before the method 300 terminates at step 340. For example, in some embodiments, in step 338, the respiratory therapy device 102 may store the therapy session inputs to the predefined preset selected in step 332.

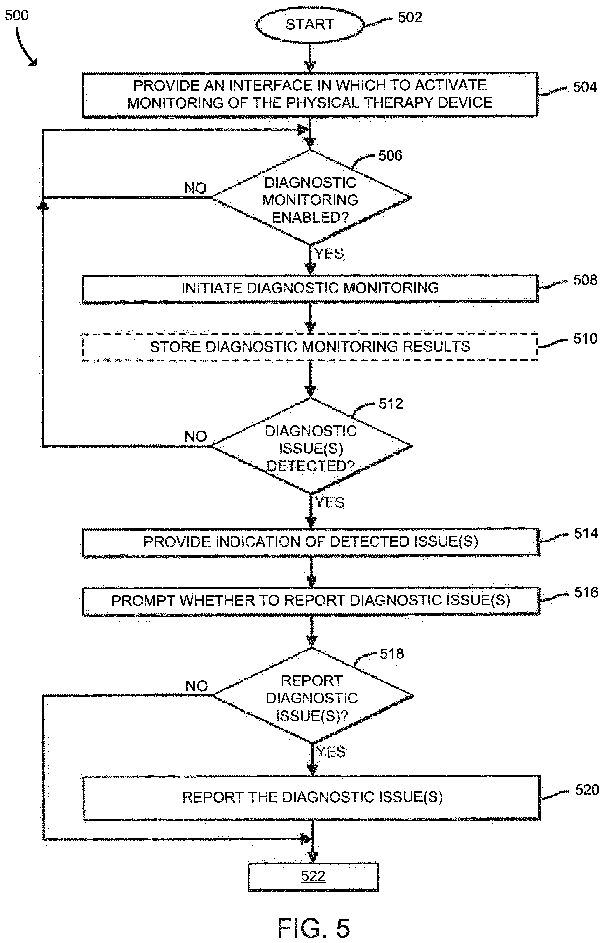

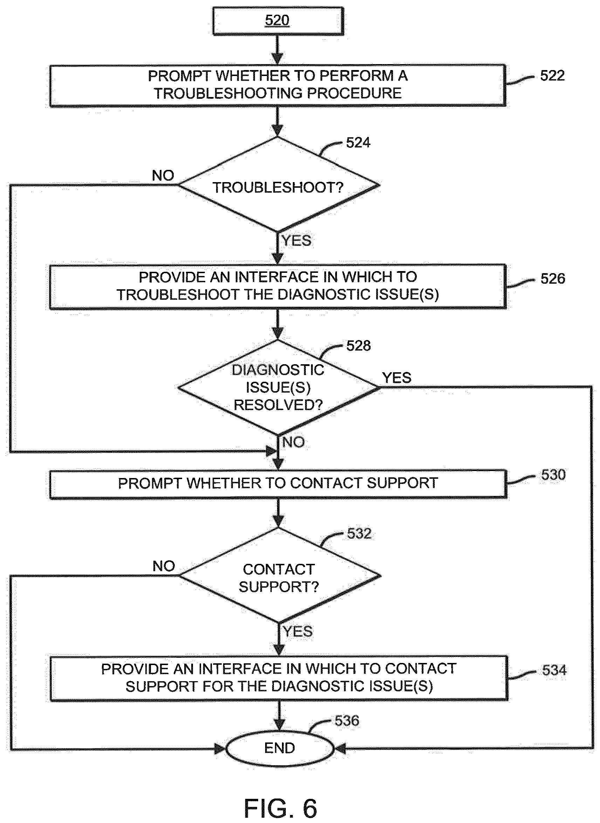

Referring now to Figs. 5 and 6, in use, the respiratory therapy device 102 and/or the computing device 104 may execute at least a portion of a method 500 for diagnostic monitoring of the respiratory therapy device 102. While at least portions of the method 500 may be performed by the respiratory therapy device 102 and/or the computing device 104, the method 500 will be described herein in the context of being performed solely by the respiratory therapy device 102 to preserve clarity of the description. As described previously, diagnostic monitoring may include checking, or otherwise testing or verifying, the functionality, settings, and/or other measurable characteristics of the respiratory therapy device 102

The method 500 starts at step 502, in which diagnostic monitoring of the respiratory therapy device 102 is initiated, which may be as a result of a user input, execution of an application, or any other action configured to initiate an option to perform diagnostic monitoring of the respiratory therapy device 102. In step 504, the respiratory therapy device 102 provides an interface in which to activate monitoring of the respiratory therapy device 102. As described previously, the interface may include a number of input/output devices, such as a display (e.g., a touchscreen display), one or more buttons, speakers, microphone, etc.

In step 506, the respiratory therapy device 102 determines whether diagnostic monitoring has been enabled. If so, the method 500 proceeds to step 508, wherein the respiratory therapy device 102 initiates the diagnostic monitoring of the respiratory therapy device 102. In some embodiments, in step 510, the respiratory therapy device 102 may store results of the diagnostic monitoring initiated in step 508 in a storage medium local to the respiratory therapy device 102 (e.g., the memory 114 or the data storage device 116).

In step 512, the respiratory therapy device 102 determines whether one or more diagnostic issues have been detected as a result of the diagnostic monitoring. If so, the method 500 proceeds to step 514, wherein the respiratory therapy device 102 provides an indication of the detected issues, such as may be indicated via a visual indicator (e.g., a display) and/or an audible indicator (e.g., a speaker). In some embodiments, the respiratory therapy device 102 may be configured to automatically report any or all of the diagnostic issues detected. Accordingly, in such embodiments, the respiratory therapy device 102 may be preconfigured with contact information for which to transmit the diagnostic issues detected. Additionally, in such embodiments, the method 500 may advance directly to step 520 (i.e., skip steps 516 and 518), which is described below.

In step 516, the respiratory therapy device 102 prompts the user whether to report the diagnostic issues detected in step 512. It should be appreciated that, as described previously, the prompt presented to the user (e.g., the prompt in step 516 and the prompts presented in subsequent steps of the method 500) may be presented to the user via an input/output device of the respiratory therapy device 102. In step 518, the respiratory therapy device 102 determines whether to report the diagnostic issue(s), such as may be determined from an input received as a result of the prompt presented in step 516. If not, the method advances to step 522, which is described below; otherwise, the method 500 advances to step 520, wherein the respiratory therapy device 102 provides an interface in which to report the diagnostic issues.

In step 522, as shown in Fig. 6, the respiratory therapy device 102 prompts the user whether to perform a troubleshooting procedure of the detected diagnostic issues. In step 522, the respiratory therapy device 102 determines whether to perform the troubleshooting procedure, such as may be determined from an input received as a result of the prompt presented in step 522. If not, the method advances to step 530, which is described below; otherwise, the method 500 advances to step 526, wherein the respiratory therapy device 102 provides an interface in which to troubleshoot detected diagnostic issues. To do so, the respiratory therapy device 102 may have locally stored troubleshooting steps which may be presented to the user via an output device of the respiratory therapy device 102, for example. Additionally or alternatively, in some embodiments, the respiratory therapy device 102 may retrieve the troubleshooting steps from the remote computing device 108.

In step 528, the respiratory therapy device 102 determines whether the detected diagnostic issues have been resolved. If so, the method 500 proceeds to step 536, wherein the method 500 terminates; otherwise, the method 500 advances to step 530, wherein the respiratory therapy device 102 prompts the user whether to contact support. If not, the method 500 proceeds to step 536, wherein the method 500 terminates; otherwise, the method 500 advances to step 534, wherein the respiratory therapy device 102 provides an interface in which to contact support for the detected diagnostic issues before the method 500 proceeds to step 536 and the method 500 terminates.

In some embodiments, the respiratory therapy device 102 may be preconfigured with contact information of a support entity such that the respiratory therapy device 102 may initiate contact with the support entity using the contact information. For example, the respiratory therapy device 102 may be preconfigured with a telephone number, a fax number, an email address, a uniform resource locator (URL), etc., of a manufacturer of the respiratory therapy device 102, an administrator of the respiratory therapy device 102, a physician of the user, etc.

As described previously, it should be appreciated that one of more functions described in the illustrative method 500 may be performed by the computing device 104. In such embodiments, the respiratory therapy device 102 and the computing device 104 may establish a communication channel (e.g., the communication channel 142 of Fig. 1) in which to transfer data therebetween and the computing device 104 may then utilize data received from the respiratory therapy device 102 to perform such functions.

It should be further appreciated that, in some embodiments, the methods 300 and 500 may be embodied as various instructions stored on a computer-readable media, which may be executed the processor 110, 130, the communication circuitry 118, 138, and/or other components of the respiratory therapy device 102 and/or the computing device 104 to cause the respiratory therapy device 102 and/or the computing device 104 to perform the methods 300 and 500. The computer-readable media may be embodied as any type of media capable of being read by the respiratory therapy device 102 and/or the computing device 104 including, but not limited to, the memory 114, 134, the data storage device 116, 136, other memory or data storage devices of the respiratory therapy device 102 and/or the computing device 104, portable media readable by a peripheral device of the respiratory therapy device 102 and/or the computing device 104, and/or other media.

Based on the foregoing, it should be appreciated that it is within the scope of the present disclosure for a patient or caregiver to monitor diagnostics of the respiratory therapy device 102 from a portable phone (e.g., cell phone, smart phone, etc.). The patient or caregiver is able to check on battery life and check on life and/or usage of mechanical components of the respiratory therapy device 102 such as mechanical percussor pods of a hoseless HFCWO system or an air pulse generator of a pneumatic HFCWO system. Alarms from the respiratory therapy device 102 are relayed to the phone of the patient or caregiver. The phone of the patient or caregiver also may be used to control the respiratory therapy device 102 according to this disclosure. In such embodiments, the phone corresponds to computing device 104 of Fig. 1.

With regard to embodiments of the HFCWO system that use mechanical percussors, such as voice coil driven mechanical percussors or solenoid driven mechanical percussors, mounted on a garment such as a vest, the phone communicates wirelessly with control circuitry on the vest such as via Bluetooth technology. A software application on the phone results in the display of a graphical user interface (GUI) on the phone to divide the therapy into different pieces. Thus, length of time of operation, voice coil or solenoid amplitude, and voice coil or solenoid frequency can be specified on the phone GUI and communicated to the circuitry of the respiratory therapy device 102, in this instance, a vest having mechanical percussors. Once the foregoing parameters are saved in the phone application, these constitute a preset that can be recalled and used in future therapy sessions. It should be appreciated that the frequency and/or amplitude may be programmed to change to different values at specified time intervals and these values are stored as part of the preset therapy parameters.

According to this disclosure, the respiratory therapy device 102 itself may have a learn function to track the user's inputs such as amplitude and frequency at various intervals over a therapy cycle. If the user liked the manner in which a particular therapy session was programmed, the user may press a preset button to enter the cycle of parameters from the therapy session into memory of the respiratory therapy device 102. Device 102 may have, for example, four buttons that each corresponds to a particular one of four stored, but different, preset therapy sessions. The user later selects a desired one of the four buttons to signal the respiratory therapy device 102 to operate according to the previously stored therapy parameters associated with the selected preset. By storing the therapy session as a preset, the user does not need to undertake the burden of adjusting the frequency and amplitude of operation of the respiratory therapy device 102 in real-time during subsequent therapy sessions. However, the user has the option of adjusting the preset therapy parameters during a current therapy session and then storing the adjusted therapy parameters as a new preset, thereby permitting users to perfect or optimize their individual therapy session parameters over time.

Although certain illustrative embodiments have been described in detail above, variations and modifications exist within the scope of this disclosure as described and as defined in the following claims.

## Claims

1. A respiratory therapy system comprising a respiratory therapy device (102) having at least one therapy administration device (122), wherein the respiratory therapy device (102) is configured to receive an input usable by the therapy administration device (122) to administer a therapy session, wherein the input comprises a total number of therapy cycles to be performed during the therapy session and a plurality of therapy parameters for each of the therapy cycles that are adjusted in real-time by a user during the therapy session,
**characterized in that** the respiratory therapy device (102) is configured to save the therapy cycles and the therapy parameters of the therapy session as a preset of the respiratory therapy device (102), wherein the preset is capable of initiating the administration of the therapy session at a future point in time based on the saved therapy cycles and the saved therapy parameters as adjusted by the user during the previous therapy session.

2. The respiratory therapy system of claim 1, wherein the respiratory therapy device is configured to receive an indication that the preset has been selected for administration of another therapy session based on the therapy cycles and the therapy parameters and to then administer another therapy session based on the therapy cycles and the therapy parameters saved to the preset.

3. The respiratory therapy system of either claim 1 or claim 2 further comprising a computing device (104) communicatively coupled to the respiratory therapy device, wherein the therapy cycles and the therapy parameters of the therapy session at the preset are saved at the computing device (104).

4. The respiratory therapy system of any preceding claim, wherein the therapy parameters comprises one or more of an oscillating compressive force, an oscillation frequency, an oscillation frequency sweep range, an oscillation frequency step range, a duration to perform one of the therapy cycles, a bias line pressure, an insufflation pressure, and an exsufflation pressure.

5. The respiratory therapy system of any preceding claim, wherein the respiratory therapy device is configured to update one or more therapy parameters of one or more cycles of the therapy session and to save the updated therapy parameters.

6. The respiratory therapy system of any preceding claim, further comprising a computing device communicatively coupled to the respiratory therapy device for providing the input usable by the respiratory therapy device to administer the therapy session.

7. The respiratory therapy system of claim 6, further comprising a communication channel (142) in which to transfer data between the computing device and the respiratory therapy device,.

8. The respiratory therapy system of claim 7, wherein the communication channel is a short-range wireless communication channel.

9. A respiratory therapy system of any preceding claim wherein the respiratory therapy device is configured to perform a diagnostic check, determine whether an issue has been detected during the diagnostic check, and provide an indication to an operator of a result of the diagnostic check.

10. A respiratory therapy system of claim 9, wherein the indication is transmitted to a computing device communicatively coupled to the respiratory therapy device, the computing device then using the indication to display the result of the diagnostic check.

11. A respiratory therapy system of any preceding claim wherein the respiratory therapy device and/or a computing device connected thereto includes a processor (110) and a memory (114).

12. A respiratory therapy system of any preceding claim comprising a network communication module (210) for facilitating network communication with the respiratory therapy device, a therapy administration module (220) configured to administer a therapy session and a therapy session management module (230) configured to manage inputs of therapy sessions.

13. A respiratory therapy system of claim 12 further comprising a diagnostic monitoring management module (240) configured to manage diagnostic monitoring.

14. A respiratory therapy system of either claim 12 or claim 13 wherein the modules form a portion of, or are established by, at least a processor of the respiratory therapy device.

## Patentansprüche

1. Ein Atemtherapiesystem, umfassend eine Atemtherapievorrichtung (102) mit mindestens einer Therapieverabreichungsvorrichtung (122), wobei die Atemtherapievorrichtung (102) so konfiguriert ist, dass sie eine Eingabe empfängt, die von der Therapieverabreichungsvorrichtung (122) verwendbar ist, um eine Therapiesitzung zu verabreichen, wobei die Eingabe eine Gesamtanzahl von Therapiezyklen, die während einer Therapiesitzung ausgeführt werden sollen und eine Vielzahl an Therapieparametern für jeden dieser Therapiezyklen umfasst, die in Echtzeit von einem Benutzer während der Therapiesitzung angepasst werden,
**dadurch gekennzeichnet, dass** die Atemtherapievorrichtung (102) so konfiguriert ist, dass sie die Therapiezyklen und die Therapieparameter der Therapiesitzung als eine Voreinstellung der Atemtherapievorrichtung (102) speichert, wobei die Voreinstellung die Verabreichung der Therapiesitzung zu einem zukünftigen Zeitpunkt, auf der Basis der gespeicherten Therapiezyklen und der gespeicherten Therapieparameter, wie sie vom Benutzer während der vorherigen Therapiesitzung eingestellt wurden, einleiten kann.

2. Das Atemtherapiesystem nach Anspruch 1, wobei die Atemtherapievorrichtung so konfiguriert ist, dass sie einen Hinweis empfängt, dass die Voreinstellung für die Verabreichung einer weiteren Therapiesitzung auf der Basis der Therapiezyklen und der Therapieparameter ausgewählt wurde, und dann eine weitere Therapiesitzung auf der Basis der Therapiezyklen und der Therapieparameter, die in den Voreinstellungen gespeichert sind, verabreicht.

3. Das Atemtherapiesystem nach entweder Anspruch 1 oder Anspruch 2, weiter umfassend ein Datenverarbeitungsgerät (104), das kommunikativ an die Atemtherapievorrichtung gekoppelt ist, wobei die Therapiezyklen und die Therapieparameter der Therapiesitzung bei der Voreinstellung auf dem Datenverarbeitungsgerät (104) gespeichert werden.

4. Das Atemtherapiesystem nach einem der vorstehenden Ansprüche, wobei die Therapieparameter eine oder mehrere von einer schwingenden Druckkraft, eine Schwingungsfrequenz, einen Sweepbereich für die Schwingungsfrequenz, einen Schrittbereich für die Schwingungsfrequenz, eine Dauer für die Durchführung eines der Therapiezyklen, einen Vorspannleitungsdruck, einen Einblasdruck und einen Ausblasdruck umfassen.

5. Das Atemtherapiesystem nach einem der vorstehenden Ansprüche, wobei die Atemtherapievorrichtung so konfiguriert ist, dass ein oder mehrere Therapieparameter der ein oder mehreren Zyklen der Therapiesitzung aktualisiert werden und dass die aktualisierten Therapieparameter gespeichert werden.

6. Das Atemtherapiesystem nach einem der vorstehenden Ansprüche, weiter umfassend ein Datenverarbeitungsgerät, das kommunikativ an die Atemtherapievorrichtung gekoppelt ist, um die Eingabe bereitzustellen, die von der Atemtherapievorrichtung verwendbar ist, um die Therapiesitzung zu verabreichen.

7. Das Atemtherapiesystem nach Anspruch 6, weiter umfassend einen Kommunikationskanal (142), in welchem Daten zwischen dem Datenverarbeitungsgerät und der Atemtherapievorrichtung übertragen werden.

8. Das Atemtherapiesystem nach Anspruch 7, wobei der Kommunikationskanal ein drahtloser Kommunikationskanal mit geringer Reichweite ist.

9. Ein Atemtherapiesystem nach einem der vorstehenden Ansprüche, wobei die Atemtherapievorrichtung so konfiguriert ist, dass sie eine Diagnoseprüfung durchführen kann; bestimmen kann, ob ein Problem während der Diagnoseprüfung erkannt wurde, und einen Hinweis zum Ergebnis der Diagnoseprüfung an den Bediener bereitstellen kann.

10. Ein Atemtherapiesystem nach Anspruch 9, wobei der Hinweis an ein Datenverarbeitungsgerät, das kommunikativ an die Atemtherapievorrichtung gekoppelt ist, übertragen wird und das Datenverarbeitungsgerät den Hinweis verwendet, um das Ergebnis der Diagnoseprüfung anzuzeigen.

11. Ein Atemtherapiesystem nach einem der vorstehenden Ansprüche, wobei die Atemtherapievorrichtung und/oder ein Datenverarbeitungsgerät, das damit verbunden ist, einen Prozessor (110) und einen Speicher (114) beinhaltet.

12. Ein Atemtherapiesystem nach einem der vorstehenden Ansprüche, umfassend ein Netzwerkkommunikationsmodul (210), um die Netzwerkkommunikation mit der Atemtherapievorrichtung zu ermöglichen, ein Therapieverabreichungsmodul (220), das so konfiguriert ist, dass es eine Therapiesitzung verabreicht und ein Therapiesitzungsverwaltungsmodul (230), das so konfiguriert ist, dass es die Eingaben von Therapiesitzungen verwaltet.

13. Ein Atemtherapiesystem nach Anspruch 12, weiter umfassend ein Verwaltungsmodul zur Diagnoseüberwachung (240), das zur Verwaltung der Diagnoseüberwachung konfiguriert ist.

14. Ein Atemtherapiesystem nach entweder Anspruch 12 oder Anspruch 13, wobei die Module einen Teil von mindestens einem Prozessor der Atemtherapievorrichtungeinen bilden oder aus mindestens einem Prozessor der Atemtherapievorrichtung bestehen.

## Revendications

1. Système de thérapie respiratoire comprenant un dispositif de thérapie respiratoire (102) ayant au moins un dispositif d'administration de thérapie (122), dans lequel le dispositif de thérapie respiratoire (102) est configuré pour recevoir une entrée utilisable par le dispositif d'administration de thérapie (122) pour administrer une séance de thérapie, dans laquelle l'entrée comprend un nombre total de cycles de thérapie à effectuer pendant la séance de thérapie et une pluralité de paramètres thérapeutiques pour chacun des cycles de thérapie qui sont réglés en temps réel par un utilisateur lors de la séance de thérapie,
**caractérisé en ce que** le dispositif de thérapie respiratoire (102) est configuré pour enregistrer les cycles de thérapie et les paramètres thérapeutiques de la séance de thérapie en tant que pré-réglage du dispositif de thérapie respiratoire (102), dans lequel le pré-réglage est capable d'initier l'administration de la séance de thérapie à un futur moment donné en fonction des cycles de thérapie enregistrés et des paramètres thérapeutiques enregistrés, tel qu'adaptés par l'utilisateur lors de la précédente séance de thérapie.

2. Système de thérapie respiratoire selon la revendication 1, dans lequel le dispositif de thérapie respiratoire est configuré pour recevoir une indication que le pré-réglage a été sélectionné pour l'administration d'une autre séance de thérapie en fonction des cycles de thérapie et des paramètres thérapeutiques et pour ensuite administrer une autre séance de thérapie en fonction des cycles de thérapie et des paramètres thérapeutiques enregistrés dans le pré-réglage.

3. Système de thérapie respiratoire selon l'une de la revendication 1 ou de la revendication 2 comprenant en outre un dispositif informatique (104) couplé en communication au dispositif de thérapie respiratoire, dans lequel les cycles de thérapie et les paramètres thérapeutiques de la séance de thérapie dans le pré-réglage sont enregistrés dans le dispositif informatique (104).

4. Système de thérapie respiratoire selon l'une quelconque des revendications précédentes, dans lequel les paramètres de thérapie comprennent un ou plusieurs parmi une force compressive oscillante, une fréquence d'oscillation, une portée de balayage de fréquence d'oscillation, un intervalle d'étape de fréquence d'oscillation, une durée pour effectuer l'un des cycles de thérapie, une pression de fluide sollicitée, une pression d'insufflation, et une pression d'exsufflation.

5. Système de thérapie respiratoire selon l'une quelconque des revendications précédentes, dans lequel le dispositif de thérapie respiratoire est configuré pour mettre à jour un ou plusieurs paramètres thérapeutiques d'un ou plusieurs cycles de la séance de thérapie et pour enregistrer les paramètres thérapeutiques mis à jour.

6. Système de thérapie respiratoire selon l'une quelconque des revendications précédentes, comprenant en outre un dispositif informatique couplé en communication au dispositif de thérapie respiratoire pour fournir l'entrée utilisable par le dispositif de thérapie respiratoire pour administrer la séance de thérapie.

7. Système de thérapie respiratoire selon la revendication 6, comprenant en outre un canal de communication (142) pour transférer des données entre le dispositif informatique et le dispositif de thérapie respiratoire.

8. Système de thérapie respiratoire selon la revendication 7, dans lequel le canal de communication est un canal de communication sans fil à courte portée.

9. Système de thérapie respiratoire selon l'une quelconque des revendications précédentes, dans lequel le dispositif de thérapie respiratoire est configuré pour effectuer une vérification de diagnostic ; déterminer si un problème a été détecté pendant la vérification de diagnostic, et fournir une indication à un opérateur d'un résultat de la vérification de diagnostic.

10. Système de thérapie respiratoire de la revendication 9, dans lequel l'indication est transmise à un dispositif informatique couplé en communication au dispositif de thérapie respiratoire, le dispositif informatique utilisant ensuite l'indication pour afficher le résultat de la vérification de diagnostic.

11. Système de thérapie respiratoire selon l'une quelconque des revendications précédentes, dans lequel le dispositif de thérapie respiratoire et/ou un dispositif informatique connecté à celui-ci inclu(en)t un processeur (110) et une mémoire (114).

12. Système de thérapie respiratoire selon l'une quelconque des revendications précédentes, comprenant un module de communication en réseau (210) pour faciliter la communication en réseau avec le dispositif de thérapie respiratoire, un module d'administration de thérapie (220) configuré pour administrer une séance de thérapie et un module de gestion de séance de thérapie (230) configuré pour gérer les entrées des séances de thérapie.

13. Système de thérapie respiratoire selon la revendication 12, comprenant en outre un module de gestion de suivi de diagnostic (240) configuré pour gérer le suivi du diagnostic.

14. Système de thérapie respiratoire selon l'une de la revendication 12 ou de la revendication 13, dans lequel les modules forment une partie de, ou sont établis par, au moins un processeur du dispositif de thérapie respiratoire.
